# DEMANDE DE BREVET EUROPEEN

(11) **EP 3 878 942 A1**
(43) Date de publication de la demande: **15.09.2021**
(21) Numéro de dépôt: 21159832.1
(22) Date de dépôt: 01.03.2021
(51) Int. Cl.: C12M 3/06, C12M 1/00, C12M 1/12

(54) **PROCÉDÉ DE PERFUSION MICRO-FLUIDIQUE D'UN SPHÉROÏDE ET DISPOSITIF ADAPTÉ POUR METTRE EN OEUVRE LEDIT PROCÉDÉ**

(30) Priorité: 09.03.2020 FR 2002290
(71) Demandeur: Commissariat à l'énergie atomique et aux énergies alternatives, 75015 Paris (FR)
(72) Inventeur: QUINTARD, Clément, 38054 GRENOBLE Cedex 09 (FR); ACHARD, Jean-Luc, 38054 GRENOBLE Cedex 09 (FR); FOUILLET, Yves, 38054 GRENOBLE Cedex 09 (FR)
(74) Mandataire: INNOV-GROUP

(57) **Abrégé**

L'invention concerne un procédé de perfusion micro-fluidique d'un sphéroïde, mis en œuvre dans un dispositif micro-fluidique qui comporte une cavité (151) de piégeage hydrodynamique dudit sphéroïde, ledit procédé comportant les étapes suivantes :
- Première injection d'un gel (20) contenant ledit sphéroïde dans le réseau micro-fluidique (10),
- Piégeage hydrodynamique dudit sphéroïde dans la cavité (151) de piégeage du réseau micro-fluidique,
- Deuxième injection d'un fluide non miscible avec ledit gel dans ledit réseau micro-fluidique pour chasser le gel présent dans le réseau, à l'exception de la cavité (151) de piégeage,
- Réticulation du gel (20) présent autour du sphéroïde, dans la cavité (151) de piégeage,
- Troisième injection d'un milieu de culture (22) dans ledit réseau micro-fluidique pour perfuser le sphéroïde fixé dans son environnement gélifié, localisé dans la cavité (151) de piégeage.

## Description

### Domaine technique de l'invention

La présente invention se rapporte à un procédé de perfusion micro-fluidique d'un sphéroïde.

L'invention concerne également le dispositif micro-fluidique configuré pour mettre en œuvre ledit procédé.

### Etat de la technique

L'invention s'inscrit dans le domaine de la perfusion de cellules, dont l'objectif est de proposer des modèles de cultures cellulaires tridimensionnels qui reproduisent au mieux, grâce à la micro-fluidique, l'environnement in vivo d'un organe.

La demande de brevet WO2019/010587A1 **(D1)** décrit principalement la création d'agrégats de cellules, dans un dispositif doté de plusieurs micro-puits.

Il existe également des solutions de culture d'agrégats de cellules. Les agrégats se présentent généralement sous la forme de sphéroïdes. Ces solutions consistent à piéger les sphéroïdes dans des micro-puits, à apporter les nutriments nécessaires aux sphéroïdes piégés et à surveiller le comportement des sphéroïdes lors de la culture. C'est le cas par exemple dans les demandes de brevets **n°**US2016/304823A1**,** US2016/097028A1**,** US2014/227784A1 et US2013/217064A1**.** La demande de brevet FR3079524A1 décrit pour sa part le principe de réalisation d'une plaque de micro-puits.

La demande de brevet FR2452285 s'intéresse quant à elle à l'encapsulation de cellules viables à l'intérieur d'une membrane.

Par ailleurs, il existe également des solutions de perfusion microfluidique de sphéroïdes. La perfusion micro-fluidique permet d'apporter les nutriments nécessaires aux cellules sur le long terme en renouvelant continument le milieu de culture, contrairement au modèle classique de culture en puits.

Dans une première approche, ces solutions de perfusion micro-fluidique consistent à figer le sphéroïde dans un gel et à le perfuser par l'intermédiaire de deux canaux latéraux dans lesquels circule le milieu de culture. De telles solutions sont par exemple décrites dans les publications suivantes :
- Nashimoto, Y., Hayashi, T., Kunita, I., Nakamasu, A., Torisawa, Y. S., Nakayama, M., ... & Yokokawa, R. (2017). Integrating perfusable vascular networks with a three-dimensional tissue in a microfluidic device. Integrative Biology, 9(6), 506-518*.*
- Van Duinen, V., Zhu, D., Ramakers, C., van Zonneveld, A. J., Vulto, P., & Hankemeier, T. (2019). Perfused 3D angiogenic sprouting in a high-throughput in vitro platform. Angiogenesis, 22(1), 157-165*.*

Une deuxième approche propose une localisation du sphéroïde grâce à un système de piégeage hydrodynamique. Le sphéroïde est injecté à travers une entrée fluidique et se retrouve piégé à un endroit choisi par le concepteur du dispositif micro-fluidique. Un flux continu peut alors être appliqué pour maintenir le sphéroïde sous perfusion. De telles solutions sont décrites dans les publications suivantes :
- Nourmohammadzadeh, M., Lo, J. F., Bochenek, M., Mendoza-Elias, J. E., Wang, Q., Li, Z. & Wang, Y. (2013). Microfluidic array with integrated oxygenation control for real-time live-cell imaging : effect of hypoxia on physiology of microencapsulated pancreatic islets. Analytical chemistry, 85(23), 11240-11249.
- Ruppen, J., Cortes-Dericks, L., Marconi, E., Karoubi, G., Schmid, R. A., Peng, R. & Guenat, O. T. (2014). A microfluidic platform for chemoresistive testing of multicellular pleural cancer spheroids. Lab on a Chip, 14(6), 1198-1205.

Ces solutions antérieures de perfusion micro-fluidique ne sont cependant pas satisfaisantes pour les raisons suivantes :
- Elles ne peuvent pas être automatisées. Le gel porteur du sphéroïde est injecté manuellement à l'aide d'une pipette et c'est à l'opérateur de déterminer le moment à partir duquel il doit stopper l'injection. Cette opération est délicate car elle nécessite de trouver un compromis entre une injection trop lente qui favoriserait la réticulation du gel avant le remplissage total du canal central, et une injection trop rapide qui favoriserait le débordement du gel dans les canaux latéraux ;
- Si le sphéroïde n'est pas encapsulé dans un gel, il baigne alors directement dans le milieu de culture, ce qui ne permet pas de se rapprocher de la réalité physiologique ;
- Si le sphéroïde est encapsulé dans un gel, l'encapsulation doit être réalisée au préalable, ce qui ne permet pas d'envisager une mise en œuvre en continu qui limiterait les interventions humaines ;
- Dans ce dernier cas, la localisation du sphéroïde reste éphémère, soumise aux fluctuations de l'écoulement, ce qui ne permet pas d'envisager une vascularisation du sphéroïde.

Le but de l'invention est de proposer un procédé de perfusion micro-fluidique d'un sphéroïde qui permet de résoudre un ou plusieurs des inconvénients des solutions antérieures. Le procédé proposé permet notamment :
- De se rapprocher au mieux de la réalité physiologique de l'être vivant dont les cellules ont été prélevées ;
- De limiter les interventions humaines, de manière à être parfaitement reproductible dans le temps ;
- De pouvoir être mis en œuvre de manière automatisée ;

### Exposé de l'invention

Ce but est atteint par un procédé de perfusion micro-fluidique d'un sphéroïde, mis en œuvre dans un dispositif micro-fluidique qui comporte :
- Un circuit micro-fluidique principal connecté entre ledit point d'entrée micro-fluidique et ledit point de sortie micro-fluidique, le circuit micro-fluidique principal comportant au moins un canal central comprenant un étranglement formant une cavité de piégeage hydrodynamique,
- Un circuit micro-fluidique secondaire connecté sur le circuit micro-fluidique principal, en parallèle dudit étranglement, ledit étranglement étant configuré pour que sa section résultante après occultation par ledit sphéroïde induise une perte de charge à travers ledit canal central qui est plus importante que la perte de charge présente dans le circuit micro-fluidique secondaire,

Ledit procédé comprenant les étapes suivantes :
- Première injection d'un gel contenant ledit sphéroïde dans le réseau micro-fluidique,
- Piégeage hydrodynamique dudit sphéroïde dans la cavité de piégeage du réseau micro-fluidique,
- Deuxième injection d'un fluide non miscible avec ledit gel dans ledit réseau micro-fluidique pour chasser le gel présent dans le réseau, à l'exception de la cavité de piégeage,
- Réticulation du gel présent autour du sphéroïde, dans la cavité de piégeage,
- Troisième injection d'un milieu de culture dans ledit réseau micro-fluidique pour perfuser le sphéroïde fixé dans son environnement gélifié, localisé dans la cavité de piégeage.

Selon une particularité, le fluide non-miscible avec le gel est l'air.

Selon une autre particularité, le gel est composé d'un mélange de fibrinogène et de thrombine, d'un mélange de fibrinogène, de collagène et de thrombine, de collagène pur ou d'un hydrogel synthétique.

Selon une autre particularité, chaque étape d'injection est réalisée par pression et/ou dépression.

Selon une variante de réalisation, le procédé comporte une étape d'addition de cellules endothéliales dans le gel pour réaliser une vascularisation du sphéroïde.

Selon une autre variante de réalisation, le procédé comporte une étape d'addition de cellules endothéliales dans le milieu de culture pour réaliser une vascularisation du sphéroïde.

L'invention concerne également le dispositif micro-fluidique de perfusion d'un sphéroïde destiné à mettre en œuvre le procédé tel que défini ci-dessus, ledit dispositif comprenant :
- Une entrée micro-fluidique, une sortie micro-fluidique et des moyens d'injection fluidique à travers ladite entrée micro-fluidique,
- Au moins une unité micro-fluidique qui comporte :
   ∘ Un point d'entrée micro-fluidique connecté à ladite entrée micro-fluidique et un point de sortie micro-fluidique connecté à ladite sortie micro-fluidique,
   ∘ Un circuit micro-fluidique principal connecté entre ledit point d'entrée micro-fluidique et ledit point de sortie micro-fluidique, le circuit micro-fluidique principal comportant au moins un canal central comprenant un étranglement formant ladite cavité de piégeage hydrodynamique,
   ∘ Un circuit micro-fluidique secondaire connecté sur le circuit micro-fluidique principal, en parallèle dudit étranglement,
   ∘ Ledit étranglement étant configuré pour que sa section résultante après occultation par ledit sphéroïde induise une perte de charge à travers ledit canal central qui est plus importante que la perte de charge présente dans le circuit micro-fluidique secondaire.

Selon une particularité, les moyens d'injection fluidique comportent :
- Au moins un réservoir contenant un gel contenant ledit sphéroïde,
- Au moins un réservoir contenant un fluide non-miscible avec ledit gel,
- Au moins un réservoir contenant un milieu de culture.

Selon une autre particularité, les moyens d'injection fluidique comportent une unité d'injection par pression connectée sur ladite entrée micro-fluidique et/ou une unité d'injection par dépression connectée sur ladite sortie micro-fluidique.

Selon une autre particularité, le circuit micro-fluidique principal et le circuit micro-fluidique secondaire comportent des canaux micro-fluidiques à section rectangulaire.

Selon une réalisation particulière, le dispositif comporte plusieurs unités micro-fluidiques identiques reliées entre elles pour former une série, chaque unité micro-fluidique étant identifiée par un rang i dans la série, avec i allant de 1 à N et N supérieur ou égal à 2, l'unité micro-fluidique de rang i, pour i allant de 2 à N-1, ayant son entrée micro-fluidique reliée à la sortie micro-fluidique de l'unité micro-fluidique de rang i-1 et sa sortie micro-fluidique reliée à l'entrée micro-fluidique de l'unité micro-fluidique de rang i+1, l'unité micro-fluidique de rang micro-fluidique de rang 1 ayant son point d'entrée micro-fluidique connecté à ladite entrée micro-fluidique et l'unité micro-fluidique de rang N ayant son point de sortie micro-fluidique connecté à ladite sortie micro-fluidique.

Selon une particularité, les unités micro-fluidiques en série peuvent être organisées en étoile ou en serpentin.

Selon une autre particularité, le dispositif comporte un réseau fluidique auxiliaire d'alimentation et de dégagement et des moyens de contrôle de l'écoulement fluidique agencés dans ledit réseau auxiliaire et dans chaque unité micro-fluidique.

### Brève description des figures

D'autres caractéristiques et avantages vont apparaître dans la description détaillée qui suit faite en regard des dessins annexés dans lesquels :
- La figure 1 représente de manière schématique, un premier exemple de réalisation du dispositif de l'invention ;
- Les figures 2A à 2C représentent trois variantes de réalisation de moyens d'injection du dispositif de l'invention ;
- La figure 3A représente une unité micro-fluidique employée dans le composant micro-fluidique du dispositif de l'invention ;
- La figure 3B montre le piège hydrodynamique dans une en vue en trois dimensions ;
- Les figures 4A et 4B, 5A et 5B, 6A et 6B illustrent le principe du procédé de l'invention appliqué sur une unité micro-fluidique du dispositif ;
- La figure 7 montre un autre exemple de réalisation de l'unité micro-fluidique du dispositif de l'invention ;
- Les figures 8A et 8B montrent deux exemples de mise en série de plusieurs unités micro-fluidiques ;
- La figure 9 montre un autre exemple de réalisation de l'unité micro-fluidique du dispositif de l'invention ;
- La figure 10 représente une mise en série de plusieurs unités micro-fluidiques dans une autre variante de réalisation ;
- Les figures 11A et 11B représentent deux modes de fonctionnement distincts possibles de la solution représentée sur la figure 10 ;
- Les figures 12 et 13 illustrent la mise en œuvre d'un principe de vascularisation du sphéroïde, mis en œuvre à l'aide du dispositif de l'invention ;

### Description détaillée d'au moins un mode de réalisation

L'invention concerne un procédé de perfusion micro-fluidique d'un agrégat de cellules. La perfusion micro-fluidique permet d'apporter les nutriments nécessaires aux cellules sur le long terme en renouvelant le milieu de culture de manière continue.

Par agrégat de cellules, on entend selon l'invention, l'auto-assemblage d'un ou plusieurs types de cellules en trois dimensions. Un tel agrégat de cellules peut notamment s'appeler sphéroïde, organoïde, neuro-sphère. Cet agrégat peut également être un îlot de Langerhans. Dans la suite de la description, on utilisera de manière générique le terme "sphéroïde", référencé S, pour évoquer un tel agrégat, ce terme étant classiquement employé dans le domaine de la culture de cellules vivantes.

De manière non limitative, un tel sphéroïde S peut par exemple présenter un diamètre allant de quelques dizaines de µm à quelques centaines de µm.

Toutes les étapes du procédé de l'invention sont mises en œuvre au sein d'un même composant micro-fluidique 1 monobloc, par exemple réalisé sous la forme d'une carte micro-fluidique. La carte micro-fluidique peut présenter des dimensions similaires à celles d'une carte de crédit.

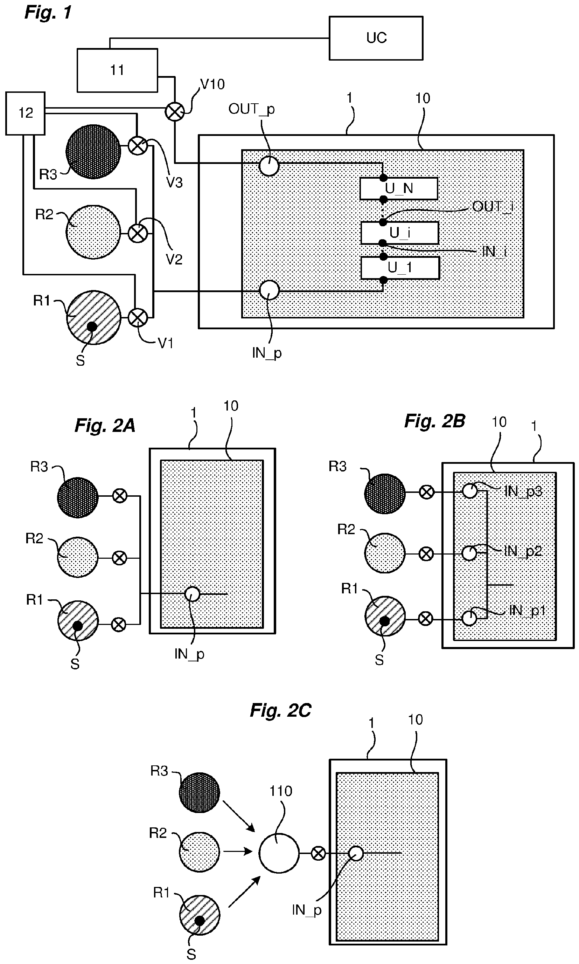

La figure 1 représente ainsi un dispositif micro-fluidique intégrant notamment le composant micro-fluidique.

Le composant micro-fluidique 1 présente des connexions micro-fluidiques et un réseau micro-fluidique 10.

Les connexions micro-fluidiques comportent au moins une entrée micro-fluidique IN_p et une sortie micro-fluidique OUT_p.

Le réseau micro-fluidique 10 est composé de canaux micro-fluidiques, réalisées dans le composant, par exemple par usinage, moulage ou autre solution technique.

Les dimensions du réseau micro-fluidique 10 sont choisies par rapport à la taille des objets biologiques étudiés, cette taille allant typiquement de quelques dizaines de micromètres à quelques centaines de micromètres. A titre d'exemple, les canaux micro-fluidiques sont de section carrée, par exemple de 400µm de côté.

Le composant micro-fluidique 1 est intégré dans un dispositif micro-fluidique.

Le dispositif micro-fluidique comporte des moyens d'injection fluidique dans ledit réseau micro-fluidique 10 du composant micro-fluidique.

Les moyens d'injection fluidique peuvent comporter une unité d'injection par pression et/ou une unité d'injection par dépression.

Comme représenté sur la figure 1, l'unité d'injection par dépression est connectée sur la sortie micro-fluidique OUT_p du composant 1 afin de permettre une injection par aspiration vers l'intérieur du réseau micro-fluidique 10 du composant 1.

Les moyens d'injection peuvent comporter au moins trois réservoirs fluidiques R1, R2, R3.

Les réservoirs fluidiques peuvent être intégrés au composant micro-fluidique 1, par exemple sous la forme de cavités creusées dans le composant. Comme sur la figure 1, les réservoirs peuvent également être externes audit composant, venant se connecter sur le réseau micro-fluidique du composant, via une connexion fluidique adaptée.

Comme illustré par la figure 2A, les réservoirs fluidiques R1, R2, R3 peuvent être connectés en parallèle sur une seule entrée IN_p micro-fluidique du composant micro-fluidique 1.

Comme illustré par la figure 2B, les réservoirs fluidiques R1, R2, R3 peuvent être connectés chacun sur une entrée micro-fluidique distincte IN_p1, IN_p2, IN_p3 du composant micro-fluidique 1.

Comme illustré par la figure 2C, l'unité d'injection 11 peut également comporter un seul bloc d'injection 110 connecté sur une seule entrée micro-fluidique du composant micro-fluidique, ledit bloc d'injection 110 étant adapté pour recevoir de manière amovible chacun des trois réservoirs R1, R2, R3.

De manière non limitative, le réservoir R1 peut contenir un gel dans lequel est placé le sphéroïde S.

Le gel peut être classiquement composé d'un mélange de fibrinogène et de thrombine, ou d'un mélange de fibrinogène, de collagène et de thrombine, la réticulation du gel étant assurée par l'interaction entre le fibrinogène et la thrombine. Le gel peut aussi être composé de collagène pur, dont la réticulation est assurée par la température. Le gel peut aussi prendre la forme d'un hydrogel synthétique. Sa réticulation peut alors être assurée par une source de lumière.

A titre d'exemple, la composition du gel utilisé peut être la suivante : fibrinogène (6,6 mg/mL), aprotinine (0,15 TIU/mL), CaCl2 (2,5 mM), thrombine (1UI/mL).

Le gel peut présenter une gamme de viscosité comprise entre celle de l'eau et une viscosité de plusieurs centaines de fois celle de l'eau (par exemple de 1 mPa.s à 1 Pa.s).

La quantité de gel injectée est typiquement de l'ordre de grandeur du volume du réseau micro-fluidique. A titre d'exemple, il peut s'agir d'un volume de gel injecté de 20µL.

Le réservoir R2 peut contenir un fluide non miscible avec le gel. Par exemple, il peut s'agir d'un fluide tel que l'air.

Le réservoir R3 peut contenir un milieu de culture adapté à la perfusion micro-fluidique du sphéroïde.

Le milieu de culture permet d'apporter les nutriments nécessaires au bon développement des cellules au sein du dispositif micro-fluidique. Il a par exemple un pH physiologique de 7,4.

Les moyens d'injection fluidique peuvent comporter plusieurs vannes micro-fluidiques Vx (avec x identifiant la vanne - V1, V2, V3 et V10 sur la figure 1 à titre d'exemple) insérées dans le réseau micro-fluidique 10 du composant 1 pour contrôler les écoulements de fluide dans ce réseau 10 ainsi qu'en sortie de chaque réservoir R1, R2, R3 pour contrôler l'écoulement fluidique en provenance de chaque réservoir. De manière non limitative, ces vannes Vx peuvent être de type pneumatique. Les moyens d'injection fluidique peuvent ainsi comporter un système d'actionnement pneumatique 12 permettant d'actionner chaque vanne Vx de manière individualisée. Chaque vanne Vx peut être à l'état ouvert dans lequel elle autorise l'écoulement fluidique ou à l'état fermé dans lequel elle bloque l'écoulement fluidique. Chaque vanne Vx peut également être commandée dans un état intermédiaire pour contrôler le débit fluidique dans le canal sur lequel elle est insérée.

De manière non limitative, les vannes peuvent être réalisées en utilisant une membrane hyper-élastique intégrée dans le composant. Soumise à une pression ou une dépression, une telle membrane est amenée à se déformer dans une cavité ou dans un canal pour contrôler l'écoulement de fluide. De telles solutions de vannes sont décrites dans les demandes de brevets EP3326717A1 et EP3085444A1**.**

Les moyens d'injection fluidique peuvent comporter une unité de commande UC. Une telle unité de commande UC peut notamment comporter un automate programmable chargé d'exécuter des commandes à destination de chaque unité d'injection 11 et dudit système d'actionnement pneumatique 12, en vue de mettre en œuvre les étapes du procédé de l'invention. L'emploi d'une unité de commande UC dans le dispositif, permet d'automatiser entièrement la mise en œuvre du procédé. L'unité de commande déroule des instructions et envoie des commandes successives aux différents moyens du dispositif, notamment pour contrôler les écoulements de fluide dans le composant 1.

Le composant micro-fluidique 1 comporte une ou plusieurs unités micro-fluidiques U_i agencées entre son entrée micro-fluidique IN_p et sa sortie micro-fluidique OUT_p. Lorsque le composant comporte plusieurs unités, celles-ci sont destinées chacune à piéger un sphéroïde distinct et à permettre une perfusion de son sphéroïde.

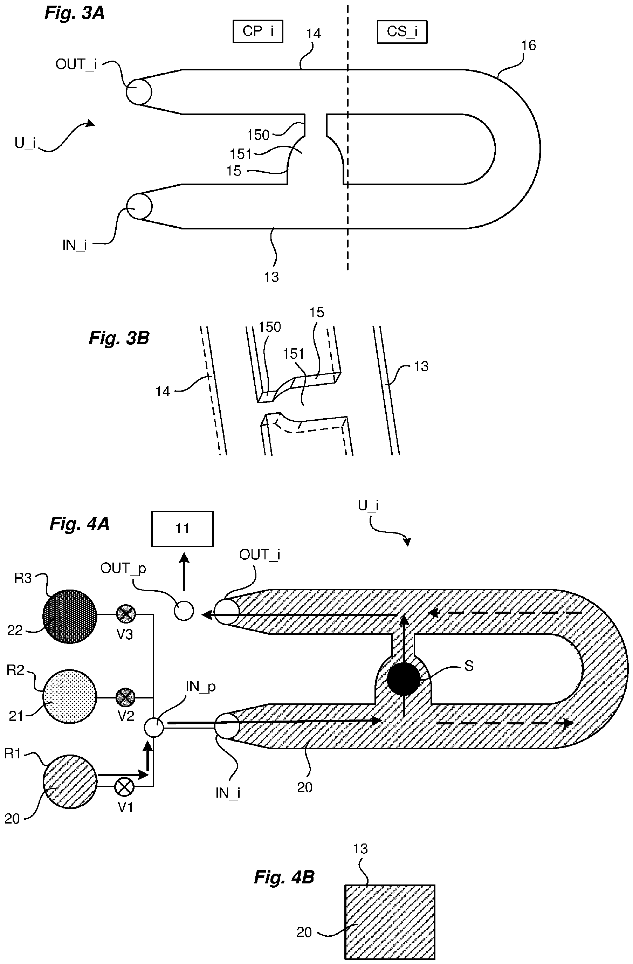

En référence à la figure 3A, chaque unité micro-fluidique U_i comporte un point d'entrée micro-fluidique IN_i et un point de sortie micro-fluidique OUT_i.

Entre son point d'entrée micro-fluidique IN_i et son point de sortie micro-fluidique OUT_i, chaque unité micro-fluidique U_i comporte un circuit micro-fluidique principal CP_i comprenant deux canaux latéraux 13, 14 et un canal central 15 reliant les deux canaux latéraux 13, 14.

Le canal central 15 du circuit micro-fluidique principal CP_i comporte un étranglement 150 ou restriction formant une cavité 151 de piégeage hydrodynamique d'un sphéroïde. La figure 3B montre un exemple de réalisation de l'étranglement 150 et donc de la cavité 151 formée.

L'unité micro-fluidique U_i comporte également un circuit micro-fluidique secondaire CS_i comportant un canal secondaire 16 connecté sur le circuit micro-fluidique principal CP_i, en parallèle de son canal central 15, de manière à former une dérivation par rapport à son étranglement 150. Sur la figure 3A, les deux circuits sont séparés par le trait en pointillés.

Selon l'invention, l'étranglement 150 est configuré pour que sa section résultante après occultation par le sphéroïde S induise une perte de charge plus importante à travers le canal central 15 que la perte de charge présente dans le circuit micro-fluidique secondaire CS_i. Plus précisément, les résistances hydrauliques du canal central 15 et du circuit micro-fluidique secondaire CS_i notées respectivement Rₚ et Rₛ sont, avant piégeage du sphéroïde, telles que Rₚ < Rₛ et, après piégeage du sphéroïde, telles que Rₚ > Rₛ. Ces inégalités garantissent le principe de fonctionnement hydrodynamique du piège. Bien entendu, plusieurs configurations et plusieurs schémas sont envisageables pour remplir ces conditions de fonctionnement.

Le principe de piégeage hydrodynamique a notamment été décrit dans la publication suivante :
- Tan, W. H., & Takeuchi, S. (2007). A trap-and-release integrated microfluidic system for dynamic microarray applications. Proceedings of the National Academy of Sciences, 104(4), 1146-1151*.*

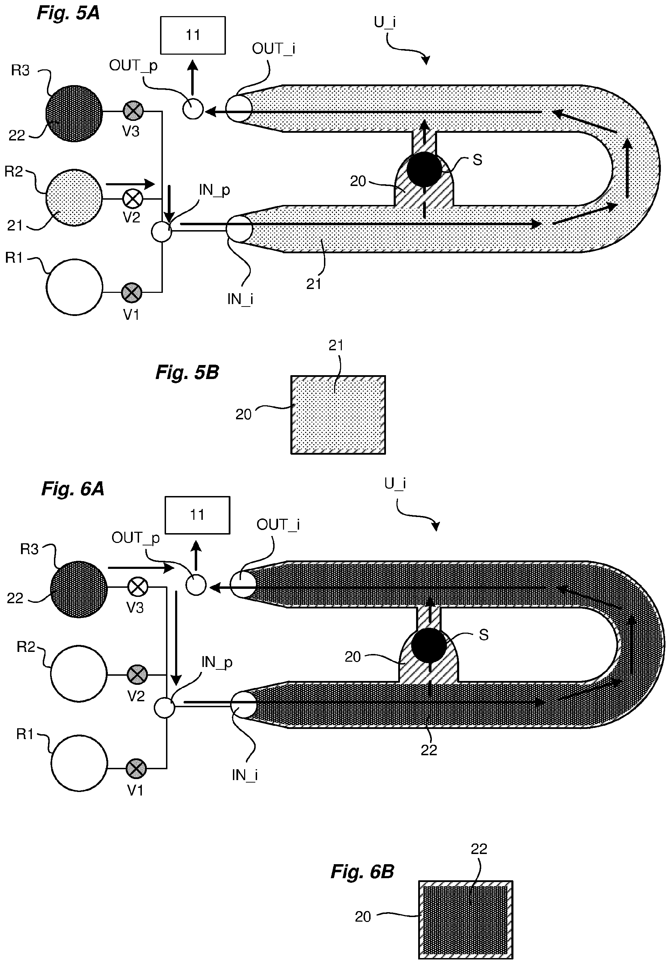

De manière non limitative, en référence aux figures 4A et 4B pour sa première phase, aux figures 5A et 5B pour sa deuxième phase et aux figures 6A et 6B pour sa troisième phase, le procédé de l'invention peut être mis en œuvre à l'aide d'un dispositif qui présente la configuration suivante :
- Une seule unité micro-fluidique (référencée U_i) ;
- Trois réservoirs R1, R2, R3 connectés en parallèle sur une seule entrée micro-fluidique IN_p du composant micro-fluidique ;
- Trois vannes V1, V2, V3 distinctes agencées en sortie de chaque réservoir pour contrôler l'écoulement des réservoirs ;
- Une unité d'injection 11 par dépression, par exemple composée d'un pousse seringue connecté à la sortie micro-fluidique du composant ;

Les figures 4B, 5B et 6B sont des vues en coupe transversale de l'un des deux canaux latéraux 13, 14 du circuit micro-fluidique principal.

### Figures 4A et 4B :

Les vannes V2 et V3 sont fermées et la vanne V1 est ouverte. L'actionnement de l'unité d'injection 11 par dépression permet d'aspirer le gel 20 porteur du sphéroïde S présent dans le réservoir vers l'intérieur du réseau micro-fluidique 10.

Lors de l'injection, le gel 20 circule préférentiellement (flèches en traits pleins) à travers le circuit micro-fluidique principal CP_i entraînant le sphéroïde S dans l'étranglement 150.

A l'issue de l'injection, le sphéroïde S vient occulter l'étranglement 150 et se loger dans la cavité 151, permettant au composant 1 de se trouver dans la situation hydrodynamique décrite ci-dessus dans laquelle la section résultante au niveau de l'étranglement 150, après occultation par le sphéroïde S, induit une perte de charge plus importante à travers le canal central 15 du circuit micro-fluidique principal CP_i que la perte de charge présente dans le circuit micro-fluidique secondaire CS_i. L'écoulement se fait dès lors majoritairement à travers le circuit micro-fluidique secondaire CS_i protégeant ainsi le sphéroïde S d'un fort cisaillement.

### Figures 5A et 5B :

Les vannes V1 et V3 sont fermées et la vanne V2 est ouverte. L'actionnement de l'unité d'injection 11 par dépression permet d'injecter le fluide non miscible avec le gel dans le réseau 10. Ce fluide peut être de l'air 21. Le gel 20 présent dans les canaux latéraux 13, 14 du circuit micro-fluidique principal CP_i et présent dans le canal secondaire 16 du circuit micro-fluidique secondaire CS_i est ainsi chassé par l'injection d'air.

Par capillarité, il subsiste cependant du gel 20 sur les parois des canaux latéraux 13, 14 du circuit micro-fluidique principal CP_i et du canal secondaire 16 ainsi que dans la cavité 151 autour du sphéroïde S, engendrant ainsi une interface gel/air le long des canaux micro-fluidiques, ce qui permet au sphéroïde de se localiser dans la cavité 151, le sphéroïde étant alors fixé dans le gel 20. Cette interface est représentée vue en coupe sur la figure 5B.

Le gel 20 réticule après quelques minutes. La configuration finale est adaptée à une perfusion micro-fluidique du sphéroïde S qui se retrouve fixé dans le gel 20, logé dans la cavité 151 et accessible pour perfusion par les canaux latéraux 13, 14 du circuit micro-fluidique principal CP_i.

### Figures 6A et 6B :

La dernière étape peut alors être mise en œuvre en injectant le milieu de culture 22 dans le réseau micro-fluidique 10. Les vannes V1 et V2 sont fermées et la vanne V3 est ouverte. L'actionnement de l'unité d'injection 11 par dépression permet d'injecter le milieu de culture 22 dans le réseau 10. Le milieu de culture 22 présent dans le réservoir R3 est constamment oxygéné et est habituellement injecté à un faible débit pendant plusieurs jours (le temps de la culture du sphéroïde S souhaité par l'opérateur).

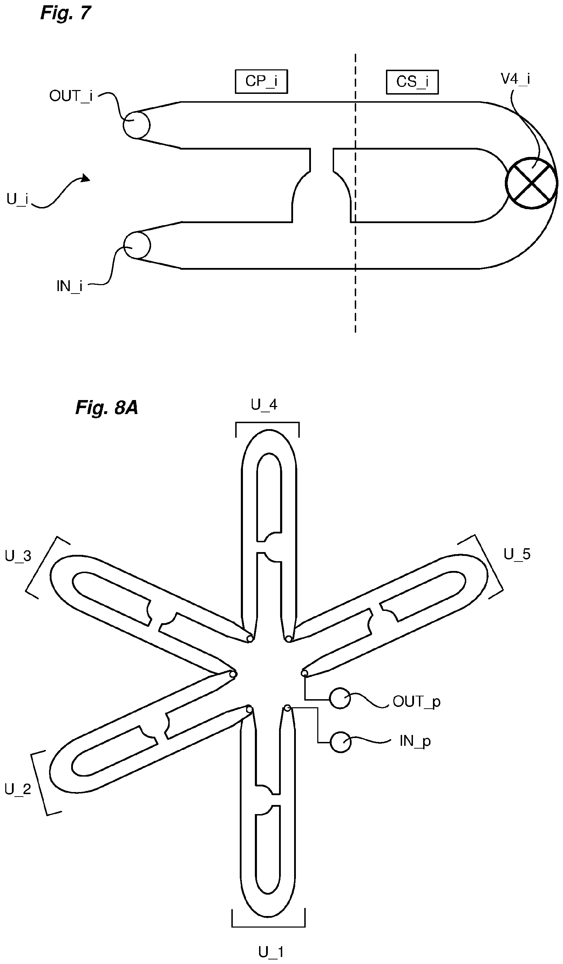

En référence à la figure 7, il est possible de prévoir des moyens de contrôle de l'écoulement fluidique à travers le sphéroïde S lors de la perfusion (troisième étape ci-dessus). Plus précisément, comme le fluide peut s'écouler via le circuit micro-fluidique principal CP_i et via le circuit micro-fluidique secondaire CS_i, il peut être souhaitable de contrôler l'écoulement dans chacune de ces deux branches. Ceci peut être réalisé en plaçant une vanne V4 dans le circuit micro-fluidique secondaire CS_i. Lorsque la vanne V4 est fermée, l'écoulement est alors forcé de se faire à travers le circuit micro-fluidique principal CP_i, et donc à travers la cavité 151 dans laquelle le sphéroïde S gélifié est piégé. Dans tous les cas, l'agencement du dispositif et le procédé de l'invention permettent de perfuser efficacement le sphéroïde S piégé dans la cavité 151 traversante, via les canaux latéraux 13, 14, et le canal central 15.

De manière avantageuse, comme indiqué ci-dessus, le dispositif peut comporter plusieurs unités micro-fluidiques U_i reliées en série entre l'entrée micro-fluidique IN_p du composant et sa sortie micro-fluidique OUT_p. Les unités micro-fluidiques en série sont par exemple toutes identiques. La mise en série permet ainsi de disposer des pièges les uns à la suite des autres et ainsi de pouvoir étudier plusieurs sphéroïdes S biologiques au sein d'un même composant micro-fluidique.

Comme indiqué sur la figure 1, dans une série, les unités micro-fluidiques peuvent être identifiées par un rang i, avec i allant de 1 à N, N étant supérieur ou égal à 2.

Le point d'entrée micro-fluidique IN_1 de l'unité micro-fluidique U_i de rang 1 est relié à l'entrée micro-fluidique IN_p du composant 10.

Le point de sortie micro-fluidique OUT_N de l'unité micro-fluidique U_N de rang N est relié à la sortie micro-fluidique OUT_p du composant.

Pour l'unité micro-fluidique U_i de rang i, lorsque i est compris entre 2 et N-1, son point d'entrée micro-fluidique IN_i est relié au point de sortie micro-fluidique OUT_i-1 de l'unité U_i-1 de rang i-1 et son point de sortie micro-fluidique OUT_i est relié au point d'entrée micro-fluidique IN_i+1 de l'unité U_i+1 de rang i+1.

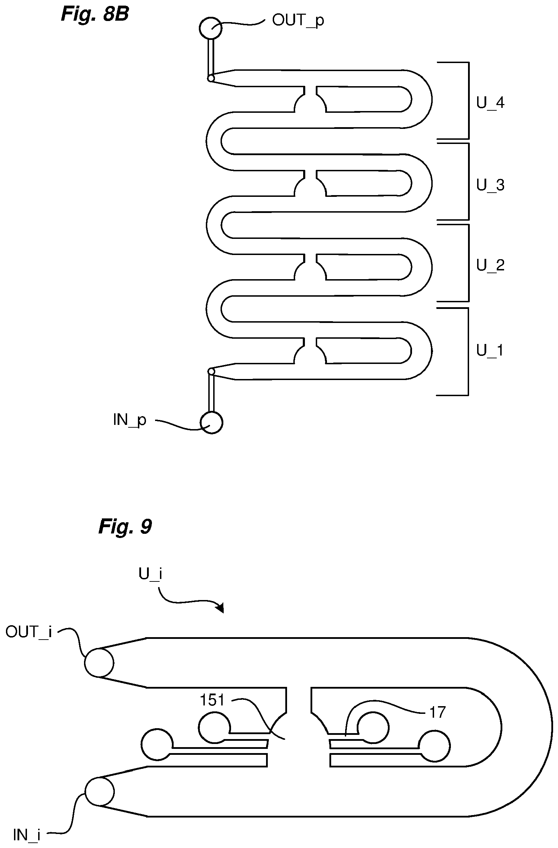

Les figures 8A et 8B représentent deux configurations de mise en série de plusieurs unités micro-fluidiques, selon les principes évoqués ci-dessus.

Sur la figure 8A, les unités micro-fluidiques sont mises en série en suivant une configuration en étoile.

Sur la figure 8B, les unités micro-fluidiques sont mises en série en suivant une configuration en serpentin.

Dans ces deux configurations, le principe de l'invention s'applique de manière identique à celui décrit ci-dessus pour une seule unité micro-fluidique (figures 4A à 6B). Le piégeage du sphéroïde S dans la cavité de la première unité U_1 de la série induit une forte perte de charge, permettant à un deuxième sphéroïde d'être acheminé vers la cavité de la deuxième unité micro-fluidique U_2 et ainsi de suite jusqu'à la dernière unité micro-fluidique U_N de la série, permettant ainsi à chaque unité micro-fluidique d'accueillir un sphéroïde distinct dans sa cavité.

Bien entendu, il faut comprendre que d'autres configurations de mise en série peuvent tout à fait être envisagées.

Ensuite les autres étapes décrites ci-dessus pour une seule unité micro-fluidique s'appliquent de manière identique à plusieurs unités en série.

La figure 9 illustre pour sa part l'ajout d'un système de perfusion double au niveau de chaque unité micro-fluidique U_i. Ce système se compose de canaux 17 débouchant directement dans la cavité 151 pour permettre une perfusion directe et indépendante du sphéroïde S piégé dans la cavité 151. Cette solution permet également de réaliser une perfusion sur le long terme à travers les canaux latéraux 13, 14 de l'unité micro-fluidique puis de perfuser les sphéroïdes de manière individualisée, sans recourir à un système de vannes et de pouvoir analyser les sécrétions du sphéroïde perfusé par ce système auxiliaire.

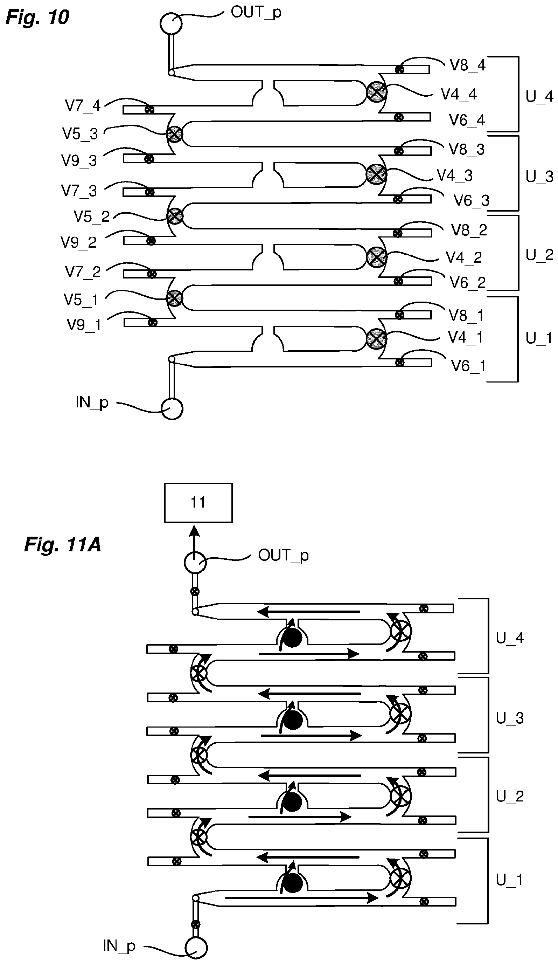

En outre, dans un dispositif à plusieurs unités micro-fluidiques mises en série, il peut s'avérer pertinent d'étudier les sphéroïdes individuellement. Pour ce faire, un système d'aiguillage peut également s'avérer nécessaire afin de pouvoir isoler chaque unité par rapport aux autres et de pouvoir acheminer les différents liquides vers des puits de sortie pour une analyse des sécrétions en aval. Dans cette solution, pour chaque unité de rang 2 à N-1, les canaux latéraux 13, 14 du circuit micro-fluidique principal CP_i peuvent être prolongés de part et d'autre. Des vannes sont également ajoutées à des endroits stratégiques. La localisation des sphéroïdes et de leur environnement se fait comme précédemment décrit à travers le circuit micro-fluidique principal CP_i de chaque unité. Sur la figure 10, on a ainsi :
- Les vannes V4_i placées dans le circuit micro-fluidique secondaire CS_i de chaque unité U_i ;
- Les vannes V5_i placées en sortie de chaque unité micro-fluidique U_i ;
- Les vannes V6_i et V7_i placées dans les deux prolongements du canal latéral 13 de chaque unité micro-fluidique U_i ;
- Les vannes V8_1 et V9_i placées dans les deux prolongements du canal latéral 14 de chaque unité micro-fluidique U_i ;

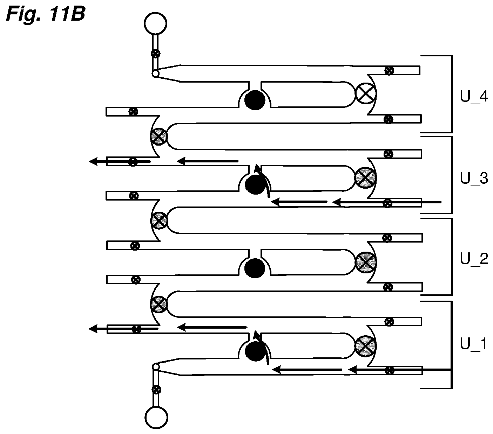

Les figures 11A et 11B illustrent deux modes de fonctionnement du dispositif représenté sur la figure 10.

Sur la figure 11A, il s'agit d'un fonctionnement classique. Des sphéroïdes sont piégés dans chaque unité micro-fluidique U_i du dispositif. Les vannes V4_i et V5_i sont ouvertes pour permettre la perfusion de l'ensemble des sphéroïdes, tandis que les vannes V6_i, V7_i, V8_i et V9_i restent fermées.

Sur la figure 11B, sur les deux unités micro-fluidiques U_1 et U3, les vannes V6_1 et V9_1 ainsi que les vannes V6_3 et V9_3 sont ouvertes, toutes les autres vannes sont fermées. Dans chacune de ces deux unités U_1 et U_3, l'écoulement est forcé à travers la cavité 151 emprisonnant leur sphéroïde S et les sécrétions du sphéroïde S peuvent ensuite être analysées en aval, en étant récupérées via le prolongement du canal 14. Ce principe peut être dupliqué afin d'analyser les sécrétions individuelles de chaque sphéroïde piégé dans une unité micro-fluidique distincte du dispositif micro-fluidique. Dans cet exemple de la figure 11B, les deux autres unités micro-fluidiques U_2 et U_4 ne sont pas actives.

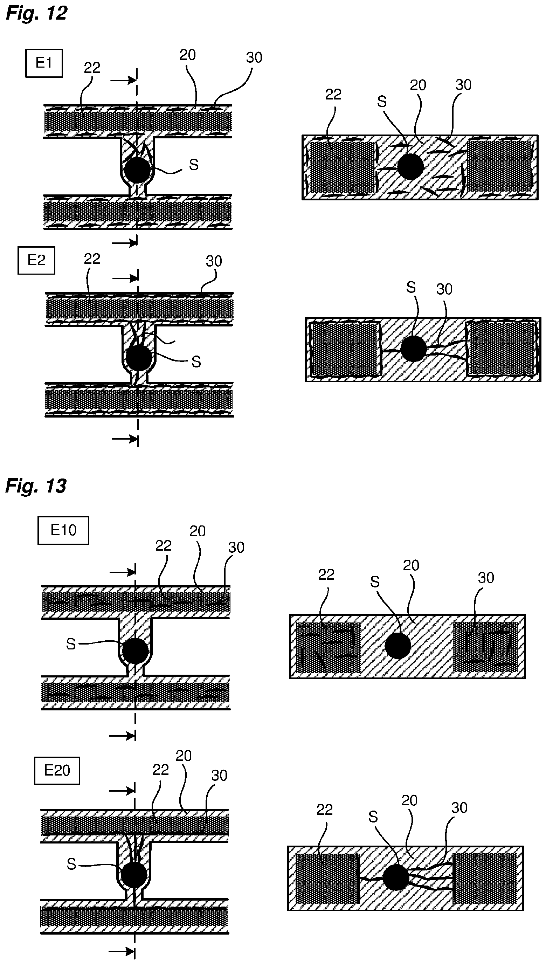

Par ailleurs, en référence aux figures 12 et 13, le dispositif de l'invention peut être employé pour mettre en œuvre une vascularisation du sphéroïde S qui a été piégé.

Sur la figure 12, la vascularisation du sphéroïde S est réalisée en injectant des cellules endothéliales 30 dans le réseau micro-fluidique. Les cellules 30 peuvent être préalablement suspendues dans le gel 20 (figure 12 - E1, vues de côté et en coupe transversale selon le trait en pointillés). Elles s'auto-organisent ensuite alors par vasculogenèse pour créer un réseau vasculaire au sein du gel 20 afin de perfuser le sphéroïde de part et d'autre (figure 12 - E2, vues de côté et en coupe transversale selon le trait en pointillés).

En référence à la figure 13, une autre façon de faire consiste à suspendre les cellules endothéliales 30 dans le milieu de culture 22 (figure 13 - E10, vues de côté et en coupe transversale selon le trait en pointillés), puis à arrêter l'écoulement et attendre quelques minutes afin que les cellules 30 sédimentent sur les parois souhaitées (figure 12 - E20, vues de côté et en coupe transversale selon le trait en pointillés). Les cellules pourront alors former un réseau vasculaire par angiogenèse en pénétrant dans le gel 20 et perfuser le sphéroïde S.

Dans les deux cas, puisqu'une fine couche de gel est déposée sur les parois du circuit micro-fluidique, aucun revêtement de surface n'est nécessaire pour une meilleure adhésion des cellules. Cette solution offre ainsi l'avantage de la rapidité.

Il faut noter que la présence d'une cavité de piégeage traversante permet de mieux perfuser le sphéroïde, de part en part via un réseau vasculaire. Par ailleurs, ce réseau vasculaire ne peut se développer que s'il bénéficie d'un support physique, c'est-à-dire l'hydrogel.

La solution de l'invention présente ainsi les avantages cités ci-dessous :
- Elle est simple, facile et rapide à mettre en œuvre, notamment grâce à son automatisation ;
- Elle est robuste et reproductible, limitant les interventions manuelles sources d'erreurs ;
- Elle ne nécessite que peu de savoir-faire, l'unité de commande permettant d'exécuter les étapes du procédé de manière entièrement automatisée ;
- Le principe de localisation du sphéroïde 4 et de son environnement 1 ne repose que sur des principes physiques (piégeage hydrodynamique, effets de capillarité) et non pas sur l'habileté de l'opérateur. Cette solution est donc facile d'utilisation y compris pour des opérateurs non experts.
- Toutes les étapes du procédé sont réalisées au sein d'un même composant micro-fluidique, celui-ci pouvant être jetable et facilement remplacé pour rentabiliser l'installation dans le temps ;

## Revendications

1. Procédé de perfusion micro-fluidique d'un sphéroïde, mis en œuvre dans un dispositif micro-fluidique qui comporte :
- Un circuit micro-fluidique principal (CP_i) connecté entre un point d'entrée micro-fluidique et un point de sortie micro-fluidique, le circuit micro-fluidique principal (CP_i) comportant au moins un canal central (15) comprenant un étranglement (150) formant une cavité (151) de piégeage hydrodynamique,
- Un circuit micro-fluidique secondaire (CS_i) connecté sur le circuit micro-fluidique principal (CP_i), en parallèle dudit étranglement (150), ledit étranglement (150) étant configuré pour que sa section résultante après occultation par ledit sphéroïde induise une perte de charge à travers ledit canal central (15) qui est plus importante que la perte de charge présente dans le circuit micro-fluidique secondaire (CS_i),
- Ledit procédé étant **caractérisé en ce qu'**il comporte les étapes suivantes :
- Première injection d'un gel (20) contenant ledit sphéroïde dans le réseau micro-fluidique (10),
- Piégeage hydrodynamique dudit sphéroïde dans la cavité (151) de piégeage du réseau micro-fluidique,
- Deuxième injection d'un fluide non miscible avec ledit gel dans ledit réseau micro-fluidique pour chasser le gel présent dans le réseau, à l'exception de la cavité (151) de piégeage,
- Réticulation du gel (20) présent autour du sphéroïde, dans la cavité (151) de piégeage,
- Troisième injection d'un milieu de culture (22) dans ledit réseau micro-fluidique pour perfuser le sphéroïde fixé dans son environnement gélifié, localisé dans la cavité (151) de piégeage.

2. Procédé selon la revendication 1, **caractérisé en ce que** le fluide non-miscible avec le gel est l'air (21).

3. Procédé selon la revendication 1 ou 2, **caractérisé en ce que** le gel est composé d'un mélange de fibrinogène et de thrombine, d'un mélange de fibrinogène, de collagène et de thrombine, de collagène pur ou d'un hydrogel synthétique.

4. Procédé selon l'une des revendications 1 à 3, **caractérisé en ce que** chaque étape d'injection est réalisée par pression et/ou dépression.

5. Procédé selon l'une des revendications 1 à 4, **caractérisé en ce qu'**il comporte une étape d'addition de cellules endothéliales (30) dans le gel (20) pour réaliser une vascularisation du sphéroïde.

6. Procédé selon l'une des revendications 1 à 4, **caractérisé en ce qu'**il comporte une étape d'addition de cellules endothéliales (30) dans le milieu de culture (22) pour réaliser une vascularisation du sphéroïde.

7. Dispositif micro-fluidique de perfusion d'un sphéroïde destiné à mettre en œuvre le procédé tel que défini dans l'une des revendications 1 à 6, ledit dispositif étant **caractérisé en ce qu'**il comporte :
- Une entrée micro-fluidique (IN_p), une sortie micro-fluidique (OUT_p) et des moyens d'injection fluidique à travers ladite entrée micro-fluidique (IN_p),
- Au moins une unité micro-fluidique (U_i) qui comporte :
∘ Un point d'entrée micro-fluidique (IN_i) connecté à ladite entrée micro-fluidique (IN_p) et un point de sortie micro-fluidique (OUT_i) connecté à ladite sortie micro-fluidique (OUT_p),
∘ Un circuit micro-fluidique principal (CP_i) connecté entre ledit point d'entrée micro-fluidique et ledit point de sortie micro-fluidique, le circuit micro-fluidique principal (CP_i) comportant au moins un canal central (15) comprenant un étranglement (150) formant ladite cavité (151) de piégeage hydrodynamique,
∘ Un circuit micro-fluidique secondaire (CS_i) connecté sur le circuit micro-fluidique principal (CP_i), en parallèle dudit étranglement (150),
∘ Ledit étranglement (150) étant configuré pour que sa section résultante après occultation par ledit sphéroïde induise une perte de charge à travers ledit canal central (15) qui est plus importante que la perte de charge présente dans le circuit micro-fluidique secondaire (CS_i).

8. Dispositif selon la revendication 7, **caractérisé en ce que** les moyens d'injection fluidique comportent :
- Au moins un réservoir (R1) contenant un gel (20) contenant ledit sphéroïde,
- Au moins un réservoir (R2) contenant un fluide non-miscible avec ledit gel (20),
- Au moins un réservoir (R3) contenant un milieu de culture (22).

9. Dispositif selon la revendication 7 ou 8, **caractérisé en ce que** les moyens d'injection fluidique comportent une unité d'injection par pression connectée sur ladite entrée micro-fluidique (IN_p) et/ou une unité d'injection (11) par dépression connectée sur ladite sortie micro-fluidique (OUT_p).

10. Dispositif selon l'une des revendications 7 à 9, **caractérisé en ce que** le circuit micro-fluidique principal (CP_i) et le circuit micro-fluidique secondaire (CS_i) comportent des canaux micro-fluidiques à section rectangulaire.

11. Dispositif selon l'une des revendications 7 à 10, **caractérisé en ce qu'**il comporte plusieurs unités micro-fluidiques (U_i) identiques reliées entre elles pour former une série, chaque unité micro-fluidique (U_i) étant identifiée par un rang i dans la série, avec i allant de 1 à N et N supérieur ou égal à 2, l'unité micro-fluidique de rang i, pour i allant de 2 à N-1, ayant son entrée micro-fluidique reliée à la sortie micro-fluidique de l'unité micro-fluidique de rang i-1 et sa sortie micro-fluidique reliée à l'entrée micro-fluidique de l'unité micro-fluidique de rang i+1, l'unité micro-fluidique de rang 1 ayant son point d'entrée micro-fluidique connecté à ladite entrée micro-fluidique et l'unité micro-fluidique de rang N ayant son point de sortie micro-fluidique connecté à ladite sortie micro-fluidique.

12. Dispositif selon la revendication 11, **caractérisé en ce que** les unités micro-fluidiques en série sont organisées en étoile.

13. Dispositif selon la revendication 11, **caractérisé en ce que** les unités micro-fluidiques en série sont organisées en serpentin.

14. Dispositif selon l'une des revendications 7 à 13, **caractérisé en ce qu'**il comporte un réseau fluidique auxiliaire d'alimentation et de dégagement et des moyens de contrôle de l'écoulement fluidique agencés dans ledit réseau auxiliaire et dans chaque unité micro-fluidique.
